# EUROPEAN PATENT APPLICATION

(11) **EP 4 349 970 A1**
(43) Date of publication of application: **10.04.2024**
(21) Application number: 22811395.7
(22) Date of filing: 27.05.2022
(51) Int. Cl.: C12N 5/079, A61K 35/30, A61L 27/38, C12N 15/63, C12Q 1/02

(54) **NEURITE OUTGROWTH PROMOTION KIT AND USE THEREFOR**

(30) Priority: 27.05.2021 JP 2021089367
(71) Applicant: Keio University, Tokyo, 108-8345 (JP)
(72) Inventor: OKANO, Hideyuki, Tokyo 160-8582 (JP); KASE, Yoshitaka, Tokyo 160-8582 (JP)
(74) Representative: Cabinet Becker et Associés
(86) International application number: PCT/JP2022/021676
(87) International publication number: WO 2022/250127

(57) **Abstract**

There are provided a neurite outgrowth promotion kit containing a γ-secretase inhibitor or a GADD45G expression vector, in which the neurite outgrowth promotion kit is used for significantly promoting neurite outgrowth of nerve cells as compared with a control, by acting on a neurosphere to induce differentiation of the neurosphere into nerve cells; a method for producing a neurosphere for treatment of spinal cord injury, which includes a step of using the above-described neurite outgrowth promotion kit on a neurosphere derived from a pluripotent stem cell; a neurosphere for treatment of spinal cord injury; and a method for selecting a neurosphere for treatment of spinal cord injury and a method for screening a neurite outgrowth-promoting agent.

## Description

### [Technical Field]

The present invention relates to a neurite outgrowth promotion kit and use therefor. More specifically, the present invention relates to a neurite outgrowth promotion kit, a method for producing a neurosphere for treatment of spinal cord injury, a neurosphere for treatment of spinal cord injury, and a method for screening a neurite outgrowth-promoting agent. Priority is claimed on Japanese Patent Application No. 2021-089367, filed May 27, 2021, the content of which is incorporated herein by reference.

### [Background Art]

Spinal cord injury is a serious injury and causes paralysis, sensory impairment, neuropathic pain, and bowel-bladder dysfunction. A large number of studies have been reported on transplanting neural stem cells or neural progenitor cells for the treatment of spinal cord injury; however, the optimal time frame for the transplantation is conceived to be the sub-acute phase after spinal cord injury. In addition, it is conceived difficult to treat spinal cord injury in the chronic phase due to changes in the intramedullary environment, such as glial scarring and cavity formation (see, for example, Non-Patent Document 1). However, most patients with spinal cord injury are in the chronic phase, and thus there is a demand for the development of a therapeutic technique that is also applicable to spinal cord injury in the chronic phase.

The inventors of the present invention previously revealed that spinal cord injury in the chronic phase can be treated by using DAPT (CAS number: 208255-80-5) on a neurosphere and then transplanting the neurosphere into a site of spinal cord injury (see, for example, Non-Patent Document 2). In addition, it was revealed that in nerve cells induced to differentiate from a neurosphere on which DAPT has been allowed to act, neurite outgrowth is promoted and the phosphorylation of p38 MAPK is increased. (see, for example, Non-Patent Document 2).

### [Citation List]

### [Non-Patent Documents]

[Non-Patent Document 1]
   Keirstead H. S., et al., Human Embryonic Stem Cell-Derived Oligodendrocyte Progenitor Cell Transplants Remyelinate and Restore Locomotion after Spinal Cord Injury, J. Neurosci., 25 (19), 4694-4705, 2005.
[Non-Patent Document 2]
   Okubo T., et al., Treatment with a Gamma-Secretase Inhibitor Promotes Functional Recovery in Human iPSC- Derived Transplants for Chronic Spinal Cord Injury, Stem Cell Reports. 11 (6), 1416-1432, 2018.

### [Summary of Invention]

### [Technical Problem]

An object of the present invention is to provide a technique for promoting neurite outgrowth of nerve cells derived from a pluripotent stem cell.

### [Solution to Problem]

The present invention includes the following aspects.
[1] A neurite outgrowth promotion kit, containing:
   a γ-secretase inhibitor; or
   a GADD45G expression vector,
   in which the neurite outgrowth promotion kit is used for promoting neurite outgrowth of nerve cells as compared with a control, by acting on a neurosphere to induce differentiation of the neurosphere into nerve cells.
[2] The neurite outgrowth promotion kit according to [1] in which the γ-secretase inhibitor is Compound 34 or DAPT.
[3] The neurite outgrowth promotion kit according to [1] or [2], further containing a dephosphorylation inhibitor of phosphorylated p38 MAPK.
[4] The neurite outgrowth promotion kit according to [3], in which the dephosphorylation inhibitor of the phosphorylated p38 MAPK is RK-682.
[5] A method for producing a neurosphere for treatment of spinal cord injury, including:
   a step of using the neurite outgrowth promotion kit according to any one of [1] to [4] on a neurosphere derived from a pluripotent stem cell.
[6] A neurosphere for treatment of spinal cord injury,
   in which an expression level of GADD45G or an amount of phosphorylated CDC25B is increased as compared with a control.
[7] A method for selecting a neurosphere for treatment of spinal cord injury, the method including:
   a step of measuring an expression level of GADD45G or an amount of phosphorylated CDC25B in a neurosphere,
   in which a case where the measured expression level of the GADD45G or the measured amount of the phosphorylated CDC25B is increased as compared with a control indicates that the neurosphere is suitable for treatment of spinal cord injury.
[8] A method for screening a neurite outgrowth-promoting agent, including:
   a step of culturing a neurosphere in a presence of a test substance; and
   a step of measuring an expression level of GADD45G or an amount of phosphorylated CDC25B in the neurosphere,
   in which a case where the expression level of the GADD45G or the amount of the phosphorylated CDC25B is increased as compared with a case where the test substance is not present indicates that the test substance is a neurite outgrowth-promoting agent.

### [Advantageous Effects of Invention]

According to the present invention, it is possible to provide a technique for promoting neurite outgrowth of nerve cells derived from a pluripotent stem cell.

### [Brief Description of Drawings]

FIGS. 1(a) to 1(g) show representative fluorescence photomicrographs showing results obtained by immunostaining βIII-tubulin in Experimental Example 1.
FIG. 2 shows a graph in which the results of FIGS. 1(a) to 1(g) are quantified.
FIG. 3 shows a graph showing results obtained by quantifying the expression level of GADD45G in Experimental Example 2.
FIGS. 4(a) to 4(c) show fluorescence photomicrographs showing representative results of immunostaining of phosphorylated p38 in Experimental Example 3.
FIG. 5 shows a graph showing results obtained by quantifying an amount of the phosphorylated p38 in Experimental Example 3.
FIG. 6 shows a graph showing results obtained by quantifying the expression level of p38 in Experimental Example 3.
FIGS. 7(a) to 7(c) show photographic images showing results of representative immunostaining of phosphorylated CDC25B in Experimental Example 4.
FIG. 8 shows a graph showing results obtained by quantifying an amount of the phosphorylated CDC25B in Experimental Example 4.
FIG. 9 shows a graph showing results obtained by quantifying the expression level of CDC25B in Experimental Example 4.
FIG. 10 shows a diagram showing a signal cascade associated with neurite outgrowth.
FIGS. 11(a) and 11(b) show fluorescence photomicrographs showing representative results of immunostaining of phosphorylated p38 in Experimental Example 5. FIG. 11(c) shows a graph showing results obtained by quantifying an amount of the phosphorylated p38 in Experimental Example 5.
FIGS. 12(a) and 12(b) show fluorescence photomicrographs showing representative results of immunostaining of phosphorylated CDC25B in Experimental Example 6. FIG. 12(c) shows a graph showing results obtained by quantifying an amount of the phosphorylated CDC25B in Experimental Example 6.
FIGS. 13(a) and 13(b) show fluorescence photomicrographs showing representative results of immunostaining of βIII-tubulin in Experimental Example 7. FIG. 13(c) shows a graph showing results obtained by quantifying the neurite length of nerve cells in Experimental Example 7.
FIGS. 14(a) and 14(b) show fluorescence photomicrographs showing representative results of immunostaining of βIII-tubulin in Experimental Example 8. FIG. 14(c) shows a graph showing results obtained by quantifying the neurite length of nerve cells in Experimental Example 8.
FIGS. 15(a) and 15(b) show fluorescence photomicrographs showing representative results of immunostaining of phosphorylated p38 in Experimental Example 9. FIG. 15(c) shows a graph showing results obtained by quantifying an amount of the phosphorylated p38 in Experimental Example 9.
FIGS. 16(a) and 16(b) show fluorescence photomicrographs showing representative results of immunostaining of phosphorylated CDC25B in Experimental Example 9. FIG. 16(c) shows a graph showing results obtained by quantifying an amount of the phosphorylated CDC25B in Experimental Example 9.
FIGS. 17(a) and 17(b) show fluorescence photomicrographs showing representative results of immunostaining of βIII-tubulin in Experimental Example 10. FIG. 17(c) shows a graph showing results obtained by quantifying the neurite length of nerve cells in Experimental Example 10.
FIG. 18 shows a cluster analysis view of all genes which have undergone variation, which is obtained by an RNA-seq analysis in Experimental Example 11.
FIGS. 19(a) and 19(b) show views showing results obtained by analyzing expression patterns of marker genes of neural stem cells and neural progenitor cells in Experimental Example 11.
FIGS. 20(a) to 20(f) show fluorescence photomicrographs showing representative results of immunostaining in Experimental Example 12.
FIG. 21(a) shows fluorescence photomicrographs showing representative results of immunostaining of phosphorylated p38 in a neurosphere derived from 201B7 cells in Experimental Example 13. FIG. 21(b) shows a graph showing results obtained by quantifying an amount of the phosphorylated p38 in the neurosphere derived from the 201B7 cells in Experimental Example 13.
FIG. 22(a) shows fluorescence photomicrographs showing representative results of immunostaining of p38 in the neurosphere derived from the 201B7 cells in Experimental Example 13. FIG. 22(b) shows a graph showing results obtained by quantifying an amount of the p38 in the neurosphere derived from the 201B7 cells in Experimental Example 13.
FIG. 23(a) shows fluorescence photomicrographs showing representative results of immunostaining of phosphorylated p38 in a neurosphere derived from 414C2 cells in Experimental Example 13. FIG. 23(b) shows a graph showing results obtained by quantifying an amount of the phosphorylated p38 in the neurosphere derived from the 414C2 cells in Experimental Example 13.
FIG. 24(a) shows fluorescence photomicrographs showing representative results of immunostaining of p38 in the neurosphere derived from the 414C2 cells in Experimental Example 13. FIG. 24(b) shows a graph showing results obtained by quantifying an amount of the p38 in the neurosphere derived from the 414C2 cells in Experimental Example 13.
FIG. 25(a) shows fluorescence photomicrographs showing representative results of immunostaining of a Tau protein in nerve cells derived from 201B7 cells in Experimental Example 14. FIG. 25(b) shows a graph showing results obtained by quantifying the neurite length of nerve cells derived from the 201B7 cell in Experimental Example 14.
FIG. 26(a) shows fluorescence photomicrographs showing representative results of immunostaining of a Tau protein in nerve cells derived from 414C2 cells in Experimental Example 14. FIG. 26(b) shows a graph showing results obtained by quantifying the neurite length of nerve cells derived from the 414C2 cells in Experimental Example 14.

### [Description of Embodiments]

### [Neurite outgrowth promotion kit]

In one embodiment, the present invention provides a neurite outgrowth promotion kit containing a γ-secretase inhibitor or a GADD45G expression vector, in which the neurite outgrowth promotion kit is used for promoting neurite outgrowth of nerve cells as compared with a control, by acting on a neurosphere to induce differentiation of the neurosphere into nerve cells. It can also be said that the neurite outgrowth promotion kit according to the present embodiment is a neurite outgrowth-promoting agent containing a γ-secretase inhibitor or a GADD45G expression vector, in which the neurite outgrowth promotion kit is used for promoting neurite outgrowth of nerve cells as compared with a control, by acting on a neurosphere to induce differentiation of the neurosphere into nerve cells.

In the present specification, neurosphere means a spherical cell aggregate that is formed by a suspension growth culture of neural stem cells or neural progenitor cells. Neural stem cell means a cell having self-replication ability and differentiation potency into a neural progenitor cell. In addition, neural progenitor cell is a cell that is undifferentiated although having been subjected to one-step differentiation from a neural stem cell, and is a cell that undergoes self-proliferation of the cell itself and finally differentiates into a nerve cell.

It is preferable that the neural stem cell or the neural progenitor cell be a cell obtained by inducing differentiation from a pluripotent stem cell. Examples of the pluripotent stem cell include an embryonic stem cell (ES cell) and an induced pluripotent stem cell (iPS cell). The pluripotent stem cell is preferably a human cell.

A method for inducing differentiation of a pluripotent stem cell into a neural stem cell or a neural progenitor cell is not particularly limited and may be a method that is usually carried out. For example, a neurosphere can be obtained by subjecting a pluripotent stem cell to suspension culture in a culture medium containing at least one of Basic fibroblast growth factor (bFGF) or Epidermal Growth Factor (EGF). In addition, a process of dissociating the obtained neurosphere into single cells, subjecting the single cells to suspension culture again in a culture medium containing bFGF, and allowing the cells to form a neurosphere again, may be repeated a plurality of times.

As will be described later in the Examples, in a case where a γ-secretase inhibitor is allowed to act on a neurosphere to induce differentiation into nerve cells, the neurite outgrowth of the nerve cells can be promoted as compared with a control.

In addition, as will be described later in Examples, in a case where a GADD45G expression vector is introduced into a neurosphere, and the neurosphere is allowed to overexpress GADD45G to induce differentiation into nerve cells, the neurite outgrowth of the nerve cells can be promoted as compared with a control. As a result, using a GADD45G expression vector on a neurosphere means introducing the GADD45G expression vector into the neurosphere. The expression vector may be a virus vector, may be a plasmid vector, or may be a transposon vector.

In the present embodiment, examples of the control include nerve cells obtained by inducing differentiation from a neurosphere on which a γ-secretase inhibitor has not been allowed to act, and nerve cells obtained by inducing differentiation from a neurosphere into which a GADD45G expression vector has not been introduced.

In the present specification, the description that neurite outgrowth is promoted refers to a case where a neurosphere is induced to differentiate into nerve cells, the number of neurites of the nerve cells is increased as compared with a control, and/or the length of the neurite is long as compared with the control.

Examples of the γ-secretase inhibitor include Compound 34 (CAS Number: 564462-36-8) and DAPT (CAS Number: 208255-80-5). Among them, Compound 34 is preferable from the viewpoint that the neurite outgrowth-promoting effect is obtained at a low concentration as compared with DAPT.

It is preferable that the neurite outgrowth promotion kit according to the present embodiment further contain a dephosphorylation inhibitor of phosphorylated p38 MAPK.

As will be described later in Examples, the inventors of the present invention revealed that in a case where Compound 34 or DAPT is allowed to act on a neurosphere, the expression level of GADD45G increases, the phosphorylation of p38 MAPK (hereinafter, may be referred to as "p38") is enhanced, and furthermore, the phosphorylation of CDC25B is enhanced. The inventors of the present invention further revealed that in a case where the dephosphorylation of phosphorylated p38 is inhibited, the phosphorylated state of p38 is maintained, the amount of phosphorylated CDC25B was significantly increased, and thus neurite outgrowth can be significantly promoted.

As a result, in a case of using a γ-secretase inhibitor and a dephosphorylation inhibitor of phosphorylated p38 in combination, it is possible to make nerve cells further improve the neurite outgrowth-promoting effect.

Examples of the dephosphorylation inhibitor of phosphorylated p38 include RK-682 (CAS number: 150627-37-5).

In a case where the neurite outgrowth promotion kit according to the present embodiment contains a γ-secretase inhibitor and a dephosphorylation inhibitor of p38, the two inhibitors may be contained in separate containers or may be mixed and contained in the same container. In a case where a γ-secretase inhibitor and a dephosphorylation inhibitor of p38 are mixed to form a composition, it can be said that the neurite outgrowth promotion kit according to the present embodiment is a neurite outgrowth-promoting composition containing a γ-secretase inhibitor and a dephosphorylation inhibitor of p38, in which the neurite outgrowth promotion kit is used for promoting neurite outgrowth of nerve cells as compared with a control, by acting on a neurosphere to induce differentiation of the neurosphere into nerve cells.

### [Method for producing neurosphere for treatment of spinal cord injury]

In one embodiment, the present invention provides a method for producing a neurosphere for treatment of spinal cord injury, which includes a step of using the above-described neurite outgrowth promotion kit on a neurosphere derived from a pluripotent stem cell.

A neurosphere for treatment of spinal cord injury can be produced by the production method according to the present embodiment. In the production method according to the present embodiment, the pluripotent stem cell, the neurosphere, and the neurite outgrowth promotion kit are the same as those described above.

In the production method according to the present embodiment, it is preferable that in a case of being added to a culture medium of the neurosphere, the γ-secretase inhibitor be allowed to act at a concentration at which the γ-secretase inhibitor effectively suppresses cell division of a neurosphere, not exhibit remarkable cytotoxicity to the neurosphere, and not form a precipitate in the culture medium.

For example, in a case where DAPT is used as the γ-secretase inhibitor, the final concentration of DAPT in a culture medium of the neurosphere is preferably 10 to 100 µM and more preferably about 10 µM.

In addition, in a case where Compound 34 is used as the γ-secretase inhibitor, the final concentration of Compound 34 in a culture medium of the neurosphere is preferably 1 to 10 µM and more preferably about 1 µM.

The period for using the γ-secretase inhibitor on the neurosphere may be 8 to 72 hours, may be 12 to 48 hours, or may be 20 to 24 hours.

In addition, in a case where RK-682 is used as a dephosphorylation inhibitor of phosphorylated p38, the final concentration of RK-682 in a culture medium of the neurosphere is preferably 10 to 20 µM and more preferably about 10 µM.

The period for using the dephosphorylation inhibitor of phosphorylated p38 on the neurosphere is preferably 10 to 100 hours and more preferably 20 to 24 hours.

The γ-secretase inhibitor and the dephosphorylation inhibitor of phosphorylated p38 may be allowed to act on the neurosphere at the same time, or the dephosphorylation inhibitor of phosphorylated p38 may be allowed to act on the neurosphere after the γ-secretase inhibitor has been allowed to act on the neurosphere.

### [Neurosphere for treatment of spinal cord injury]

In one embodiment, the present invention provides a neurosphere for treatment of spinal cord injury, in which the expression level of GADD45G or an amount of phosphorylated CDC25B is increased as compared with a control. Here, examples of the control include a neurosphere on which a γ-secretase inhibitor has not been allowed to act.

As will be described later in Examples, the inventors of the present invention revealed that in a neurosphere which has been treated with Compound 34 or DAPT, the expression level of GADD45G is significantly increased, the amount of phosphorylated p38 is significantly increased, and the amount of phosphorylated CDC25B is significantly increased, as compared with a neurosphere which has not been treated with Compound 34 or DAPT. Accordingly, in the neurosphere according to the present embodiment, neurite outgrowth is promoted in a case where the neurosphere is induced to differentiate into nerve cells.

The inventors of the present invention also revealed that in a neurosphere overexpressing GADD45G, the amount of phosphorylated p38 is significantly increased, and neurite outgrowth is significantly promoted in a case where the neurosphere is induced to differentiate into nerve cells.

Accordingly, spinal cord injury can be effectively treated by transplanting the neurosphere according to the present embodiment into a site of spinal cord injury of a patient with spinal cord injury in the chronic phase.

The expression level of GADD45G may be measured in terms of the mRNA level or may be measured in terms of the protein level. The expression level of mRNA of GADD45G can be measured by quantitative RT-PCR or the like. The expression level of the GADD45G protein can be measured by immunostaining of cells, Western blotting, or the like. In addition, the amount of phosphorylated CDC25B can be measured by immunostaining of cells, Western blotting, or the like.

It is preferable that the neurosphere according to the present embodiment be a neurosphere produced by the above-described method for producing a neurosphere for treatment of spinal cord injury.

### [Method for selecting neurosphere for treatment of spinal cord injury]

In one embodiment, the present invention provides a method for selecting a neurosphere for treatment of spinal cord injury, the method including a step of measuring the expression level of GADD45G or an amount of phosphorylated CDC25B in a neurosphere, in which a case where the measured expression level of the GADD45G or the measured amount of the phosphorylated CDC25B is increased as compared with a control indicates that the neurosphere is suitable for treatment of spinal cord injury. Here, examples of the control include a neurosphere on which a γ-secretase inhibitor has not been allowed to act.

According to the method for selecting according to the present embodiment, spinal cord injury can be effectively treated by transplanting a neurosphere shown to be suitable for treatment of spinal cord injury into a site of spinal cord injury of a patient with spinal cord injury in the chronic phase. The method for measuring the expression level of GADD45G and the method for measuring the amount of phosphorylated CDC25B are the same as those described above.

### [Method for screening neurite outgrowth-promoting agent]

In one embodiment, the present invention provides a method for screening a neurite outgrowth-promoting agent, including a step of culturing a neurosphere in the presence of a test substance and a step of measuring the expression level of GADD45G or an amount of phosphorylated CDC25B in the neurosphere, in which a case where the expression level of the GADD45G or the amount of the phosphorylated CDC25B is increased as compared with a case where the test substance is not present indicates that the test substance is a neurite outgrowth-promoting agent.

As will be described later in Examples, the inventors of the present invention revealed that in a case where a neurosphere in which the expression level of GADD45G or the amount of phosphorylated CDC25B is increased is induced to differentiate into nerve cells, the neurite outgrowth of the nerve cells can be promoted as compared with a control. Here, examples of the control include nerve cells obtained by inducing differentiation from a neurosphere on which a γ-secretase inhibitor has not been allowed to act.

Accordingly, it can also be said that a test substance that increases the expression level of GADD45G or the amount of phosphorylated CDC25B in the neurosphere is a neurite outgrowth-promoting agent in a case where the neurosphere is cultured in the presence of the test substance.

The method for measuring the expression level of GADD45G and the method for measuring the amount of phosphorylated CDC25B are the same as those described above.

The test substance is not particularly limited, and examples thereof include a natural compound library, a synthetic compound library, an existing drug library, and a metabolite library.

The test substance may be allowed to act on a neurosphere, may be allowed to act in at least a part of a step of inducing differentiation of a neurosphere into nerve cells, or may be allowed to act in the entire step of inducing differentiation of a neurosphere into nerve cells. The test substance may be allowed to act for, for example, 20 to 24 hours.

### [Examples]

Next, the present invention will be described in more detail by showing Examples; however, the present invention is not limited to Examples below.

### [Experimental Example 1]

### (Examination of neurite outgrowth-promoting effect of γ-secretase inhibitor)

Various γ-secretase inhibitors were allowed to act on neurospheres derived from human iPS cells. Subsequently, each neurosphere was induced to differentiate into nerve cells, and the neurite outgrowth was examined. The following inhibitors were used as γ-secretase inhibitors: DAPT (CAS Number: 208255-80-5), Compound 34 (CAS Number: 564462-36-8), L-685,458 (CAS Number: 292632-98-5), LY411575 (CAS number: 209984-57-6), Sulindac (CAS number: 38194-50-2, which is also an antiinflammatory drug), and Compound E (CAS number: 209986-17-4).

First, 201B7 cells, which are cells of a human iPS cell line, were differentiated into neurospheres. 3 µM SB431542 (Tocris Bioscience, catalog number: "301836-41-9") and 150 nM LDN193189 (StemRD Inc., catalog number: "1062368-24-4") were added to a culture medium of 201B7 cells, and the cells were treated for 6 days.

Subsequently, the cells were dissociated and seeded in a cell culture dish (product name: "ultralow-attachment culture dish", Corning Incorporated) at a cell density of 1 × 10⁵ cells/mL in a nerve induction medium, and then cultured for 6 days in a hypoxic and moist environment (4% O₂ and 5% CO₂) to carry out differentiation into neurospheres.

The culture medium used as the nerve induction medium was a culture medium obtained by adding a 2% B27 supplement (without vitamin A, Thermo Fisher Scientific, Inc., catalog number: "17504-044"), 20 ng/mL of FGF2 (PeproTech, Inc.), 10 µM Y27632 (Sigma-Aldrich Co., LLC, catalog number: "146986-50-7"), 1 µM retinoic acid (RA, Sigma-Aldrich Co., LLC, catalog number: "R2625-1G"), 3 µM CHIR 99021 (ReproCELL Inc., catalog number: "04 -0004"), and 10 µM SB431542 (Calbiochem, catalog number: "301836-41-9"), to an MHM medium of which the composition is shown in Table 1 below.

**[Table 1]**

| Media Hormone Mix (MHM) medium | |
|---|---|
| 10 × DMEM/F12 | 50 mL |
| 10 × Hormone mix | 50 mL |
| 200 mM L-Glutamine | 5 mL |
| Penicillin-streptomycin | 5 ml (final concentration: 0.5%) |
| 30% Glucose | 10 mL |
| 7.5% NaHCO₃ | 7.5 mL |
| 1M HEPES | 2.5 mL |
| Water | 375 mL |
| Total | 500 mL |
| 10 × Hormone mix | |
| Water | 652 mL |
| 10 × DMEM/F12 | 80 mL |
| 30% Glucose | 16 mL |
| 7.5% NaHCO₃ | 12 mL |
| 1M HEPES | 4 mL |
| Transferrin | 800 mg |
| Putrescine | 77 mg |
| Insulin | 200 mg |
| 2 mM Progesterone | 80 µL |
| 3 mM Sodium selenite | 80 µL |
| Total | 764 mL |

Subsequently, the formed neurospheres were dissociated into single cells, the single cells were passaged in a modified nerve induction medium and cultured for 6 days in a hypoxic environment (4% O₂). The culture medium used as the modified nerve induction medium was a culture medium obtained by adding a 2% B27 supplement (without vitamin A, catalog number: "17504-044", Thermo Fisher Scientific, Inc.), 20 ng/mL of FGF2 (PeproTech, Inc.), 10 µM Y27632 (catalog number: "146986-50-7", Sigma-Aldrich Co., LLC), and 1 µM retinoic acid (RA, catalog number: "R2625-1G", Sigma-Aldrich Co., LLC), to an MHM medium of which the composition is shown in Table 1 above.

Subsequently, neurospheres were cultured for 24 hours in the presence of each γ-secretase inhibitor. Each inhibitor was added to a culture medium of the neurosphere so that the final concentration was 10 µM for DAPT, 1 µM for Compound 34, 10 µM for L-685,458, 10 µM for LY411575, 10 µM for Sulindac, and 5 µM for Compound E. In addition, a control group cultured in the absence of a γ-secretase inhibitor was also prepared.

Subsequently, each neurosphere was seeded on a 48-well chamber slide (IWAKI & CO., LTD.) coated with poly-L-ornithine/fibronectin, cultured for 14 days in a culture medium containing no growth factor at 37°C in an environment of 95% air and 5% CO₂, and differentiated into nerve cells.

Subsequently, each of the obtained nerve cells was fixed with 4% paraformaldehyde in 0.1 M PBS, and each of βIII-tubulin, which is a nerve cell marker, and the Tau protein, which is frequently distributed in axons, was immunostained, and the length of the neurite was measured.

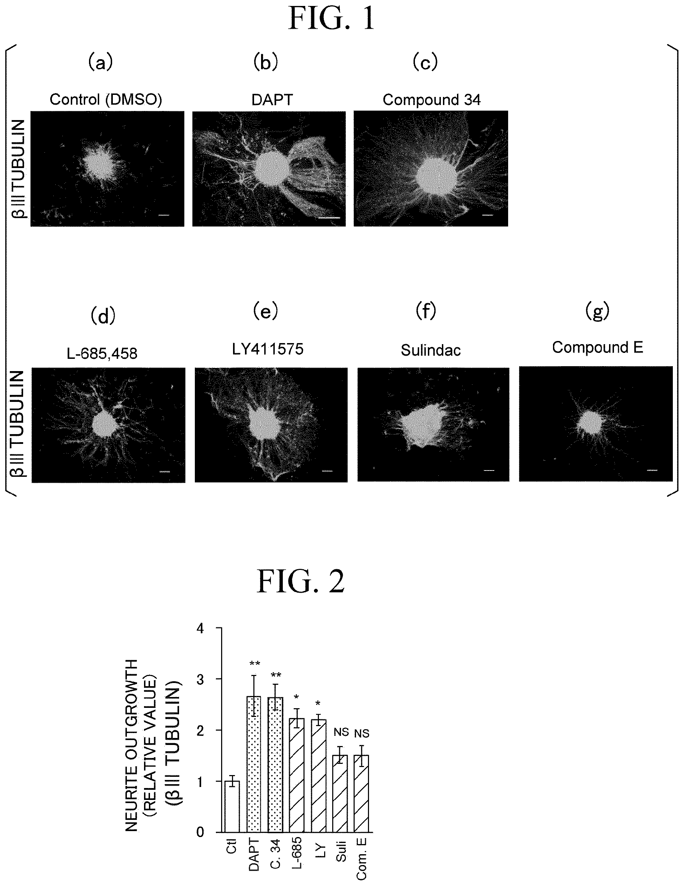

FIGS. 1(a) to 1(g) show representative fluorescence photomicrographs of each nerve cell in which βIII-tubulin was immunostained. The scale bar is 100 µm. FIG. 1(a) shows a photographic image of a control nerve cell, FIG. 1(b) shows a photographic image of a nerve cell obtained by inducing differentiation of a DAPT-treated neurosphere, FIG. 1(c) shows a photographic image of a nerve cell obtained by inducing differentiation of a Compound 34-treated neurosphere, FIG. 1(d) shows a photographic image of a nerve cell obtained by inducing differentiation of an L-685,458-treated neurosphere, FIG. 1(e) shows a photographic image of a nerve cell obtained by inducing differentiation of an LY411575-treated neurosphere, FIG. 1(f) shows a photographic image of a nerve cell obtained by inducing differentiation of a Sulindac-treated neurosphere, and FIG. 1(g) shows a photographic image of a nerve cell obtained by inducing differentiation of a Compound E-treated neurosphere.

FIG. 2 is a graph in which the results of FIGS. 1(a) to 1(g) are quantified. In FIG. 2, "Ctl" indicates the result obtained from the control, "DAPT" indicates the result obtained by the DAPT treatment, "C. 34" indicates the result obtained by Compound 34 treatment, "L-685" indicates the result obtained by the L-685,458 treatment, "LY" indicates the result obtained by the LY411575 treatment, "Suli" indicates the result obtained by the Sulindac treatment, and "Com. E" indicates the result obtained by the Compound E treatment. In addition, "**" indicates that there is a significant difference at p < 0.01, "*" indicates that there is a significant difference at p < 0.05, and "NS" indicates that there is no significant difference.

As a result, it was revealed that neurite outgrowth is particularly promoted in the DAPT-treated and Compound 34-treated groups among the γ-secretase inhibitors.

In the Tau protein immunostaining as well, the same results as in the immunostaining of βIII-tubulin were obtained, and it was revealed that the neurite outgrowth was particularly promoted in the DAPT-treated and Compound 34-treated groups.

### [Experimental Example 2]

### (Examination 1 of signal cascade associated with neurite outgrowth)

### <<Examination of expression level of GADD45G>>

Neurospheres derived from 201B7 cells, which are cells of a human iPS cell line, were prepared in the same manner as in Experimental Example 1.

In addition, neurospheres derived from 414C2 cells, which are cells of a human iPS cell line, were prepared according to the following method. First, 414C2 cells were subjected to adhesion culture for 12 days together with fibroblasts derived from a mouse embryo. Subsequently, the cells were subjected to suspension culture for 30 days to form a germ layer body. Subsequently, the aggregated cells were dissociated, cultured in a culture medium obtained by adding 20 ng/mL of FGF2 (PeproTech, Inc.) to an MHM medium of which the composition is shown in Table 1 above, and differentiated into neurospheres.

Subsequently, each neurosphere derived from iPS cells was treated with each of DAPT and Compound 34. Subsequently, one neurosphere having a diameter of about 100 µm was selected from the neurospheres in each group and subjected to an RNA-seq analysis.

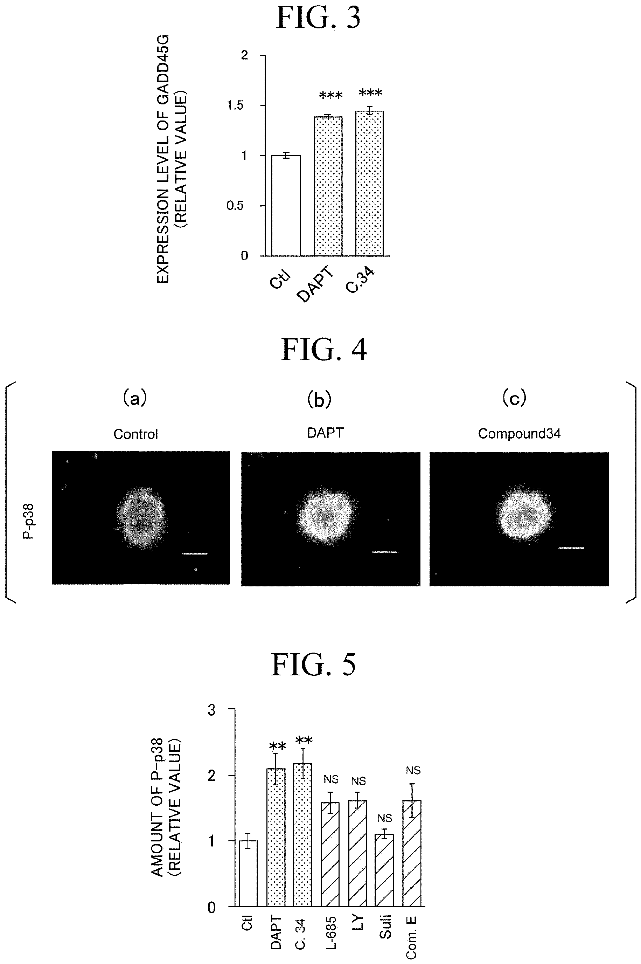

FIG. 3 is a graph showing the results obtained by treating the neurosphere derived from 201B7 cells with each of DAPT and Compound 34, carrying out the RNA-seq analysis, and quantifying the expression level of GADD45G based on transcriptome data. In FIG. 3, "Ctl" indicates the result obtained from the control, "DAPT" indicates the result obtained by the DAPT treatment, and "C. 34" indicates the result obtained by Compound 34 treatment. In addition, "***" indicates that there is a significant difference at p < 0.001.

As a result, it was revealed that the expression of GADD45G was significantly increased in both the DAPT treatment and Compound 34 treatment. The same results were also obtained regarding the neurosphere derived from 414C2 cells.

### [Experimental Example 3]

### (Examination 2 of signal cascade associated with neurite outgrowth)

### <<Examination of phosphorylated p38>>

Neurospheres derived from 201B7 cells were treated with various γ-secretase inhibitors in the same manner as in Experimental Example 1. As the γ-secretase inhibitor, DAPT, Compound 34, L-685,458, LY411575, Sulindac, and Compound E were used at the same concentrations as in Experimental Example 1. Subsequently, each neurosphere was fixed with paraformaldehyde and subjected to immunostaining of phosphorylated p38.

FIGS. 4(a) to 4(c) show fluorescence photomicrographs showing the representative results of immunostaining. FIG. 4(a) shows the result obtained from the control, FIG. 4(b) shows the result obtained by the DAPT-treated result, and FIG. 4(c) shows the result obtained by the Compound 34 treatment. In FIGS. 4(a) to 4(c), "P-p38" indicates phosphorylated p38. In addition, the scale bar is 100 µm.

FIG. 5 is a graph showing the results obtained by quantifying the amount of the phosphorylated p38 in the neurosphere which had been treated with each of the γ-secretase inhibitors. In FIG. 5, "Ctl" indicates the result obtained from the control, "DAPT" indicates the result obtained by the DAPT treatment, "C. 34" indicates the result obtained by Compound 34 treatment, "L-685" indicates the result obtained by the L-685,458 treatment, "LY" indicates the result obtained by the LY411575 treatment, "Suli" indicates the result obtained by the Sulindac treatment, and "Com. E" indicates the result obtained by the Compound E treatment. In addition, "**" indicates that there is a significant difference at p < 0.01, and "NS" indicates that there is no significant difference.

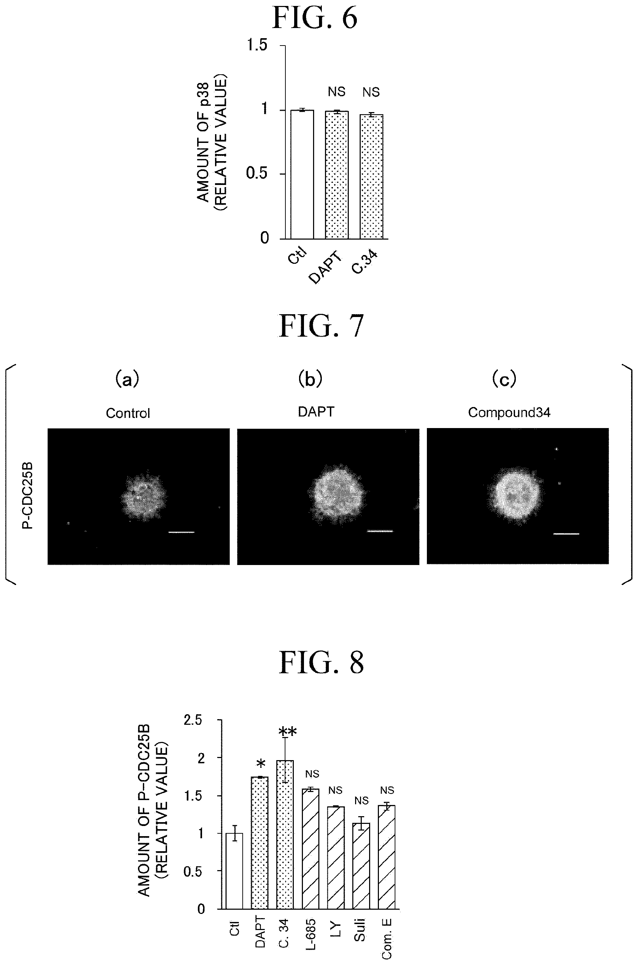

FIG. 6 is a graph showing the results obtained by treating the neurosphere derived from 201B7 cells with each of DAPT and Compound 34, carrying out the RNA-seq analysis, and quantifying the expression level of p38 based on transcriptome data. In FIG. 6, "Ctl" indicates that it is the result obtained from the control, "DAPT" indicates that it is the result obtained by the DAPT treatment, and "C. 34" indicates that it is the result obtained by Compound 34 treatment. In addition, "NS" indicates that there is no significant difference.

As a result, it was revealed that the amount of phosphorylated p38 in the neurosphere is significantly increased by the DAPT treatment or Compound 34 treatment, but the expression level of p38 is not increased.

### [Experimental Example 4]

### (Examination 3 of signal cascade associated with neurite outgrowth)

### <<Examination of phosphorylated CDC25B>>

Neurospheres derived from 201B7 cells were treated with various γ-secretase inhibitors in the same manner as in Experimental Example 1. As the γ-secretase inhibitor, DAPT, Compound 34, L-685,458, LY411575, Sulindac, and Compound E were used at the same concentrations as in Experimental Example 1. Subsequently, each neurosphere was fixed with paraformaldehyde and subjected to immunostaining of phosphorylated CDC25B.

FIGS. 7(a) to 7(c) show fluorescence photomicrographs showing the representative results obtained by the DAPT treatment and Compound 34 treatment. FIG. 7(a) shows the result obtained from the control, FIG. 7(b) shows the result of the DAPT treatment, and FIG. 7(c) shows the result of the Compound 34 treatment. In FIGS. 7(a) to 7(c), "P-CDC25B" indicates phosphorylated CDC25B. In addition, the scale bar is 100 µm.

FIG. 8 is a graph showing the results obtained by quantifying the amount of the phosphorylated CDC25B in the neurosphere which had been treated with each of the γ-secretase inhibitors. In FIG. 8, "Ctl" indicates that it is the result obtained from the control, "DAPT" indicates that it is the result obtained by the DAPT treatment, "C. 34" indicates that it is the result obtained by Compound 34 treatment, "L-685" indicates that it is the result obtained by the L-685,458 treatment, "LY" indicates that it is the result obtained by the LY411575 treatment, "Suli" indicates that it is the result obtained by the Sulindac treatment, and "Com. E" indicates that it is the result obtained by the Compound E treatment. In addition, "*" indicates p < 0.05 and indicates that there is a significant difference, "**" indicates p < 0.01 and indicates that there is a significant difference, and "NS" indicates that there is no significant difference.

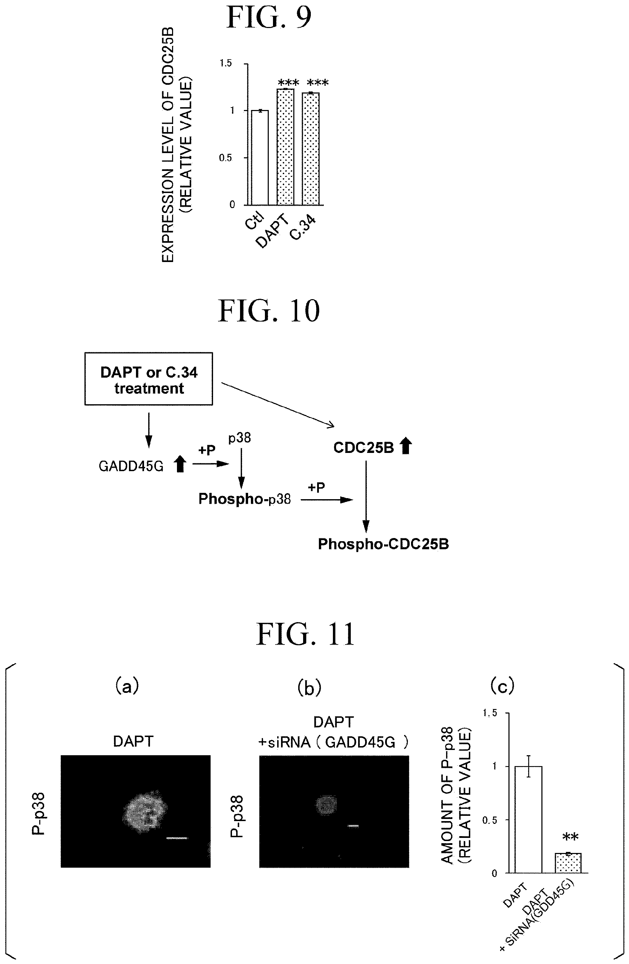

FIG. 9 is a graph showing the results obtained by treating the neurosphere derived from 201B7 cells with each of DAPT and Compound 34, carrying out the RNA-seq analysis, and quantifying the expression level of CDC25B based on transcriptome data. In FIG. 9, "Ctl" indicates that it is the result obtained from the control, "DAPT" indicates that it is the result obtained by the DAPT treatment, and "C. 34" indicates that it is the result obtained by Compound 34 treatment. In addition, "***" indicates p < 0.001 and indicates that there is a significant difference.

As a result, it was revealed that the amount of phosphorylated CDC25B in the neurosphere is significantly increased by the DAPT treatment or Compound 34 treatment. In addition, it was revealed that the expression level of CDC25B is also slightly increased.

From the above results, the inventors of the present invention hypothesized that the signal cascade shown in FIG. 10 was associated with neurite outgrowth.

### [Experimental Example 5]

### (Verification 1 of signal cascade associated with neurite outgrowth)

### <<Knockdown of GADD45G>>

It was verified whether or not the signal cascade shown in FIG. 10 was actually associated with neurite outgrowth. First, neurospheres derived from 201B7 cells, which are cells of a human iPS cell line, were prepared in the same manner as in Experimental Example 1. Subsequently, an siRNA (catalog number: "4392420", Thermo Fisher Scientific, Inc.) against GADD45G was introduced into the neurosphere. A commercially available kit (product name: "HiPerFect Transfection Reagent", catalog number: "301705", QIAGEN N.V.) was used for the introduction of siRNA. In addition, a group into which a control siRNA (catalog number: "4390844", Thermo Fisher Scientific, Inc.) had been introduced was also prepared as a control.

Subsequently, each neurosphere was cultured for 24 hours in the presence of each of DAPT and Compound 34. DAPT and Compound 34 were used at the same concentrations as in Experimental Example 1.

Subsequently, each neurosphere was fixed with paraformaldehyde and subjected to immunostaining of phosphorylated p38. FIGS. 11(a) and 11(b) show fluorescence photomicrographs showing the representative results of immunostaining. FIG. 11(a) shows the results of the control into which a control siRNA has been introduced and then the DAPT treatment has been carried out, and FIG. 11(b) shows the results obtained by introducing the siRNA against GADD45G and then carrying out the DAPT treatment. The scale bar is 100 µm. In FIGS. 11(a) and 11(b), "P-p38" indicates phosphorylated p38.

FIG. 11(c) shows a graph showing the results obtained by quantifying the amount of the phosphorylated p38 in each group. In FIG. 11(c), "DAPT" indicates that it is the result obtained from the control into which a control siRNA has been introduced and then the DAPT treatment has been carried out, and "DAPT + siRNA (GADD45G)" indicates that it is the result obtained by introducing the siRNA against GADD45G and then carrying out the DAPT treatment. In addition, "**" indicates p < 0.01 and indicates that there is a significant difference.

As a result, it was revealed that in a case of introducing the siRNA against GADD45G and then carrying out the DAPT treatment, the increase in the amount of phosphorylated p38 in the neurosphere is significantly suppressed. In addition, even in a case where Compound 34 was used, the same result as in the case where DAPT was used was obtained. That is, it was revealed that in a case of introducing the siRNA against GADD45G and then carrying out the Compound 34 treatment, the increase in the amount of phosphorylated p38 in the neurosphere is significantly suppressed.

### [Experimental Example 6]

### (Verification 2 of signal cascade associated with neurite outgrowth)

### <<Examination of effect of addition of SB203580 on phosphorylation of CDC25B>>

Neurospheres derived from 201B7 cells, which are cells of a human iPS cell line, were prepared in the same manner as in Experimental Example 1. Subsequently, each neurosphere was cultured for 24 hours in the presence of each of DAPT and Compound 34. DAPT and Compound 34 were used at the same concentrations as in Experimental Example 1. In addition, in this case, SB203580 (CAS number: 152121-47-6), which is a p38 inhibitor, was added to the culture medium at a final concentration of 20 µM. In addition, a group to which SB203580 had not been added was also prepared as a control. Subsequently, each neurosphere was fixed with paraformaldehyde and subjected to immunostaining of phosphorylated CDC25B.

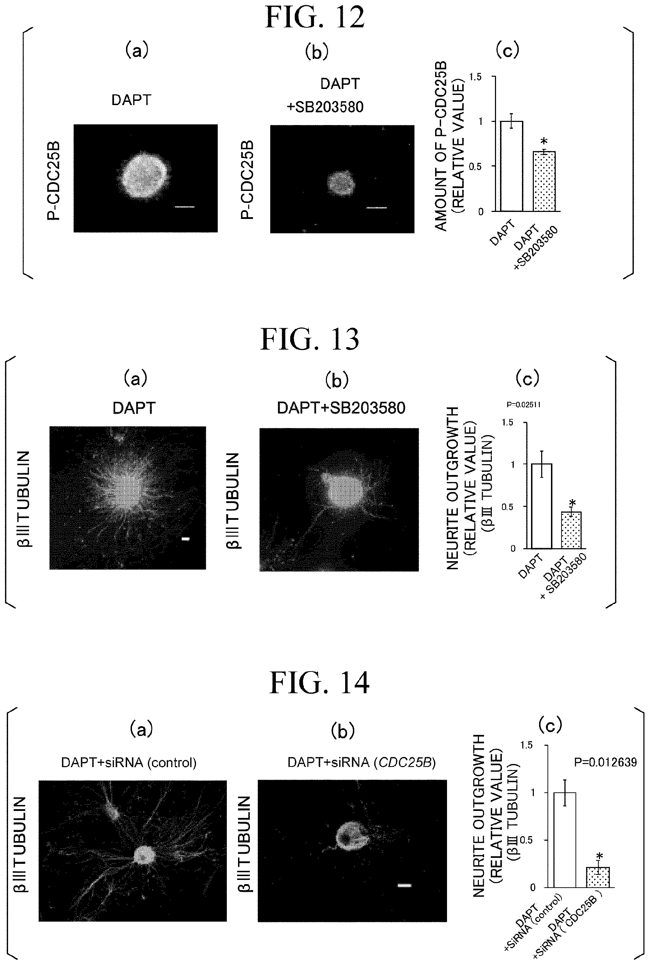

FIGS. 12(a) and 12(b) show fluorescence photomicrographs showing the representative results of immunostaining. FIG. 12(a) shows the results obtained from the control into which the DAPT treatment has been carried out in the absence of SB203580, and FIG. 12(b) shows the results obtained by carrying out the DAPT treatment in the presence of SB203580. The scale bar is 100 µm. In FIGS. 12(a) and 12(b), "P-CDC25B" indicates phosphorylated CDC25B.

FIG. 12(c) shows a graph showing the results obtained by quantifying the amount of the phosphorylated CDC25B in each group. In FIG. 12(c), "DAPT" indicates it is the result obtained from the control into which the DAPT treatment has been carried out in the absence of SB203580, and "DAPT + SB203580" indicates it is the result obtained by carrying out the DAPT treatment in the presence of SB203580. In addition, "*" indicates p < 0.05 and indicates that there is a significant difference.

As a result, it was revealed that in a case of carrying out the DAPT treatment in the presence of SB203580, the increase in the amount of phosphorylated CDC25B in the neurosphere is significantly suppressed. In addition, even in a case where Compound 34 was used, the same result as in the case where DAPT was used was obtained. That is, it was revealed that in a case of carrying out the Compound 34 treatment in the presence of SB203580, the increase in the amount of phosphorylated CDC25B in the neurosphere is significantly suppressed.

### [Experimental Example 7]

### (Verification 3 of signal cascade associated with neurite outgrowth)

### <<Examination of effect of addition of SB203580 on neurite outgrowth>>

Neurospheres derived from 201B7 cells, which are cells of a human iPS cell line, were prepared in the same manner as in Experimental Example 1. Subsequently, each neurosphere was cultured for 24 hours in the presence of each of DAPT and Compound 34. DAPT and Compound 34 were used at the same concentrations as in Experimental Example 1. In addition, in this case, SB203580 (CAS number: 152121-47-6), which is a p38 inhibitor, was added to the culture medium at a final concentration of 20 µM. In addition, a group to which SB203580 had not been added was also prepared as a control.

Subsequently, each neurosphere was seeded on a 48-well chamber slide (IWAKI & CO., LTD.) coated with poly-L-ornithine/fibronectin, cultured for 14 days in a culture medium containing no growth factor at 37°C in an environment of 95% air and 5% CO₂, and differentiated into nerve cells.

Subsequently, each of the obtained nerve cells was fixed with 4% paraformaldehyde in 0.1 M PBS, and each of βIII-tubulin which is a nerve cell marker, and the Tau protein which is frequently distributed in axons, was immunostained, and the length of the neurite was measured.

FIGS. 13(a) and 13(b) show fluorescence photomicrographs showing representative results of immunostaining of βIII-tubulin. FIG. 13(a) shows the results obtained by the control into which the DAPT treatment has been carried out in the absence of SB203580, and FIG. 13(b) shows the results obtained by carrying out the DAPT treatment in the presence of SB203580. The scale bar is 100 µm.

FIG. 13(c) shows a graph showing results obtained by quantifying the neurite length of the nerve cells in each group. In FIG. 13(c), "DAPT" indicates it is the result obtained from the control into which the DAPT treatment has been carried out in the absence of SB203580, and "DAPT + SB203580" indicates it is the result obtained by carrying out the DAPT treatment in the presence of SB203580. In addition, "*" indicates p < 0.05 and indicates that there is a significant difference.

As a result, it was revealed that in a case of carrying out the DAPT treatment in the presence of SB203580, the neurite outgrowth is significantly suppressed. In addition, even in a case where Compound 34 was used, the same result as in the case where DAPT was used was obtained. That is, it was revealed that in a case of carrying out the Compound 34 treatment in the presence of SB203580, the neurite outgrowth is significantly suppressed.

### [Experimental Example 8]

### (Verification 4 of signal cascade associated with neurite outgrowth)

### <<Knockdown of CDC25B>>

Neurospheres derived from 201B7 cells, which are cells of a human iPS cell line, were prepared in the same manner as in Experimental Example 1. Subsequently, an siRNA (catalog number: "4390824", Thermo Fisher Scientific, Inc.) against CDC25B was introduced into the neurosphere. A commercially available kit (product name: "HiPerFect Transfection Reagent", catalog number: "301705", QIAGEN N.V.) was used for the introduction of siRNA. In addition, a group into which a control siRNA (catalog number: "4390844", Thermo Fisher Scientific, Inc.) had been introduced was also prepared as a control.

Subsequently, each neurosphere was cultured for 24 hours in the presence of each of DAPT and Compound 34. DAPT and Compound 34 were used at the same concentrations as in Experimental Example 1.

Subsequently, each neurosphere was seeded on a 48-well chamber slide (IWAKI & CO., LTD.) coated with poly-L-ornithine/fibronectin, cultured for 14 days in a culture medium containing no growth factor at 37°C in an environment of 95% air and 5% CO₂, and differentiated into nerve cells.

Subsequently, each of the obtained nerve cells was fixed with 4% paraformaldehyde, and each of βIII-tubulin which is a nerve cell marker, and the Tau protein which is frequently distributed in axons, was immunostained, and the length of the neurite was measured.

FIGS. 14(a) and 14(b) show fluorescence photomicrographs showing the representative results of immunostaining. FIG. 14(a) shows the results obtained by the control into which a control siRNA has been introduced and then the DAPT treatment has been carried out, and FIG. 14(b) shows the results obtained by introducing the siRNA against CDC25B and then carrying out the DAPT treatment. The scale bar is 100 µm.

FIG. 14(c) shows a graph showing results obtained by quantifying the neurite length of the nerve cells in each group. In FIG. 14(c), "DAPT + siRNA (control)" indicates that it is the result obtained from the control into which a control siRNA is introduced and then the DAPT treatment is carried out, and "DAPT + siRNA (CDC25B)" indicates that it is the result obtained by introducing the siRNA against CDC25B and then carrying out the DAPT treatment. In addition, "*" indicates p < 0.05 and indicates that there is a significant difference.

As a result, it was revealed that in a case of introducing the siRNA against CDC25B and then carrying out the DAPT treatment, neurite outgrowth is significantly suppressed. In addition, it was revealed that the same results as in the immunostaining of βIII-tubulin were also obtained in the Tau protein immunostaining, and the neurite outgrowth is significantly suppressed in a case of introducing the siRNA against CDC25B and then carrying out the DAPT treatment.

In addition, from the results of immunostaining of βIII-tubulin and the Tau protein, it was revealed that although the differentiation into nerve cells is not inhibited, the neurite outgrowth is inhibited in a case where CDC25B is subjected to knockdown.

In addition, even in a case where Compound 34 was used, the same result as in the case where DAPT was used was obtained. That is, it was revealed that in a case of introducing the siRNA against CDC25B and then carrying out the Compound 34 treatment, neurite outgrowth is significantly suppressed.

In addition, even in a case where DAPT was used and even in a case where Compound 34 was used, the neurite outgrowth was suppressed by about 75% by the knockdown of CDC25B. From this result, CDC25B was conceived to be the most downstream effector in the signal cascade associated with neurite outgrowth.

### [Experimental Example 9]

### (Verification 5 of signal cascade associated with neurite outgrowth)

### <<Examination of effect of RK-682 on phosphorylation of p38 and CDC25B>>

It was examined whether or not the phosphorylation of CDC25B was upregulated by maintaining and promoting the phosphorylation of p38. RK-682 (CAS number: 150627-37-5) is a dephosphorylation inhibitor for tyrosine phosphorylation, which is isolated from the bacteria of the genus Streptomyces. RK-682 has an action of maintaining p38 in an activated state by suppressing the dephosphorylation of phosphorylated p38. In addition, it is said that RK-682 does not have a dephosphorylation inhibitory effect on CDC25B.

Neurospheres derived from 201B7 cells, which are cells of a human iPS cell line, were prepared in the same manner as in Experimental Example 1. Subsequently, the neurospheres were cultured for 24 hours in the presence of RK-682 at a final concentration of 20 µM. In addition, as a control, a group to which RK-682 was not added was also prepared. Subsequently, each neurosphere was fixed with paraformaldehyde and subjected to immunostaining of each of the phosphorylated p38 and the phosphorylated CDC25B.

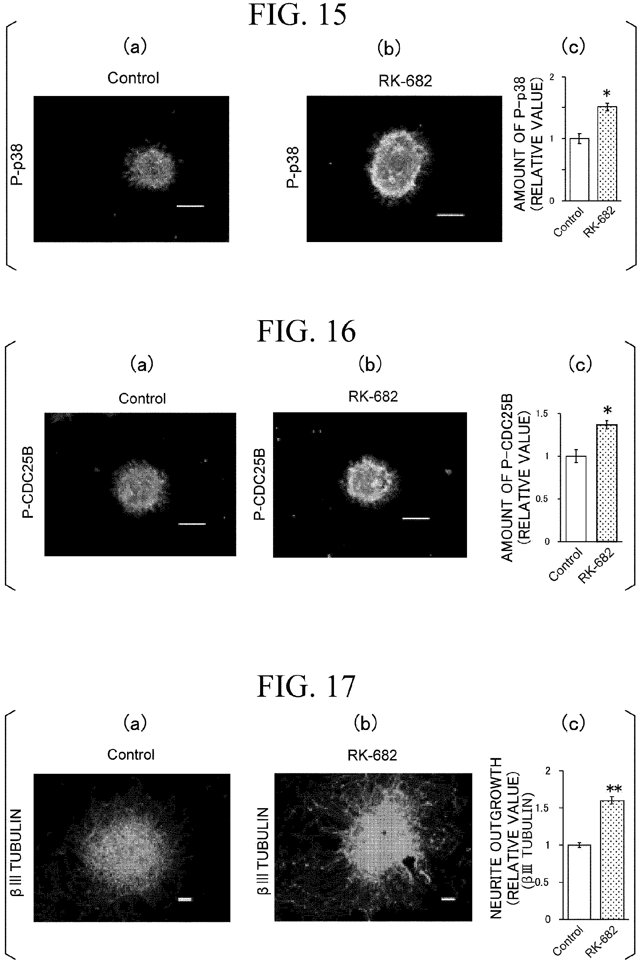

FIGS. 15(a) and 15(b) show fluorescence photomicrographs showing the representative results of immunostaining of phosphorylated p38. FIG. 15(a) shows the result obtained from the control, and FIG. 15(b) shows the result obtained by carrying out the RK-682 treatment. The scale bar is 100 µm. In FIGS. 15(a) and 15(b), "P-p38" indicates phosphorylated p38.

FIG. 15(c) shows a graph showing the results obtained by quantifying the amount of the phosphorylated p38 in each group. In FIG. 15(c), "Control" indicates that it is the result obtained from the control which has not been subjected to the RK-682 treatment, and "RK-682" indicates that it is the result obtained by carrying out the RK-682 treatment. In addition, "*" indicates p < 0.05 and indicates that there is a significant difference.

As a result, it was revealed that in a case of carrying out the RK-682 treatment, the amount of phosphorylated p38 in the neurosphere is significantly increased.

In addition, FIGS. 16(a) and 16(b) show fluorescence photomicrographs showing the representative results of immunostaining of phosphorylated CDC25B. FIG. 16(a) shows the result obtained from the control, and FIG. 16(b) shows the result obtained by carrying out the RK-682 treatment. The scale bar is 100 µm. In FIGS. 16(a) and 16(b), "P-CDC25B" indicates phosphorylated CDC25B.

FIG. 16(c) shows a graph showing the results obtained by quantifying the amount of the phosphorylated CDC25B in each group. In FIG. 16(c), "Control" indicates that it is the result obtained from the control which has not been subjected to the RK-682 treatment, and "RK-682" indicates that it is the result obtained by carrying out the RK-682 treatment. In addition, "*" indicates p < 0.05 and indicates that there is a significant difference.

As a result, it was revealed that in a case of carrying out the RK-682 treatment, the amount of phosphorylated CDC25B in the neurosphere is significantly increased.

### [Experimental Example 10]

### (Verification 6 of signal cascade associated with neurite outgrowth)

### <<Examination of effect of RK-682 on neurite outgrowth>>

Neurospheres derived from 201B7 cells, which are cells of a human iPS cell line, were prepared in the same manner as in Experimental Example 1. Subsequently, the neurospheres were cultured for 24 hours in the presence of RK-682 at a final concentration of 20 µM. In addition, as a control, a group to which RK-682 was not added was also prepared.

Subsequently, each neurosphere was seeded on a 48-well chamber slide (IWAKI & CO., LTD.) coated with poly-L-ornithine/fibronectin, cultured for 14 days in a culture medium containing no growth factor at 37°C in an environment of 95% air and 5% CO₂, and differentiated into nerve cells.

Subsequently, each of the obtained nerve cells was fixed with 4% paraformaldehyde in 0.1 M PBS, and each of βIII-tubulin which is a nerve cell marker, and the Tau protein which is frequently distributed in axons, was immunostained, and the length of the neurite was measured.

FIGS. 17(a) and 17(b) show fluorescence photomicrographs showing representative results of immunostaining of βIII-tubulin. FIG. 17(a) shows the result obtained from the control, and FIG. 17(b) shows the result obtained by carrying out the RK-682 treatment. The scale bar is 100 µm.

FIG. 17(c) shows a graph showing results obtained by quantifying the neurite length of the nerve cells in each group. In FIG. 17(c), "Control" indicates that it is the result obtained from the control which has not been subjected to the RK-682 treatment, and "RK-682" indicates that it is the result obtained by carrying out the RK-682 treatment. In addition, "**" indicates p < 0.01 and indicates that there is a significant difference.

As a result, it was revealed that in a case of carrying out the RK-682 treatment, the neurite outgrowth is significantly promoted. In addition, in the Tau protein immunostaining as well, the same results as in the immunostaining of βIII-tubulin were obtained, and it was revealed that the neurite outgrowth is significantly promoted.

From the results of Experimental Examples 5 to 9, it was demonstrated that the signal cascade associated with the neurite outgrowth, which is shown in FIG. 10, is correct. In addition, it was shown that RK-682 is a compound that promotes neurite outgrowth by utilizing the signal cascade shown in FIG. 10.

### [Experimental Example 11]

### (Analysis of gene expression pattern)

As described above, the inventors of the present invention demonstrated a signal cascade associated with the promotion of neurite outgrowth in a case where DAPT or Compound 34 is allowed to act on the neurosphere to induce differentiation into nerve cells.

Then, the comprehensive variation in the transcription levels of genes was examined by RNA-seq analysis between DAPT and Compound 34 in a case where DAPT and Compound 34 were allowed to act on neurospheres.

Neurospheres derived from 201B7 cells, which are cells of a human iPS cell line, and neurospheres derived from 414C2 cells, which are cells of a human iPS cell line, were each prepared in the same manner as in Experimental Example 1. Subsequently, each neurosphere derived from iPS cells was treated with each of DAPT and Compound 34. Subsequently, one neurosphere having a diameter of about 100 µm was selected from the neurospheres in each group and subjected to an RNA-seq analysis.

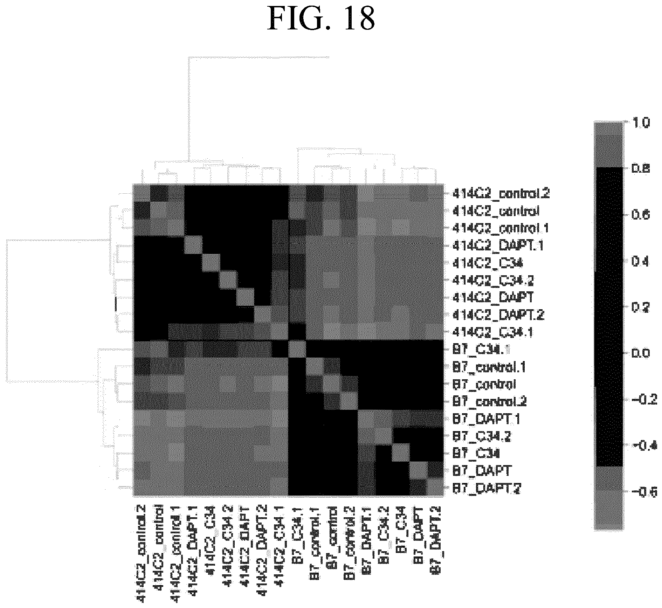

FIG. 18 is a cluster analysis view for all genes which have undergone variation, which is obtained by an RNA-seq analysis. In FIG. 18, "B7" indicates that it is the result obtained from the neurosphere derived from 201B7 cells, "414C2" indicates that it is the result obtained from the neurosphere derived from 414C2 cells, "Control" indicates that it is the result obtained from the control on which neither DAPT nor Compound 34 has been allowed to act, "DAPT" indicates that it is the result obtained by using DAPT, and "C34" indicates that it is the result obtained by using Compound 34.

As a result, in both the neurosphere derived from 201B7 cells and the neurosphere derived from 414C2 cells, it was revealed that the gene expression variation pattern after the DAPT treatment and the gene expression variation pattern after the Compound 34 treatment are in a homologous relationship in terms of the clustering.

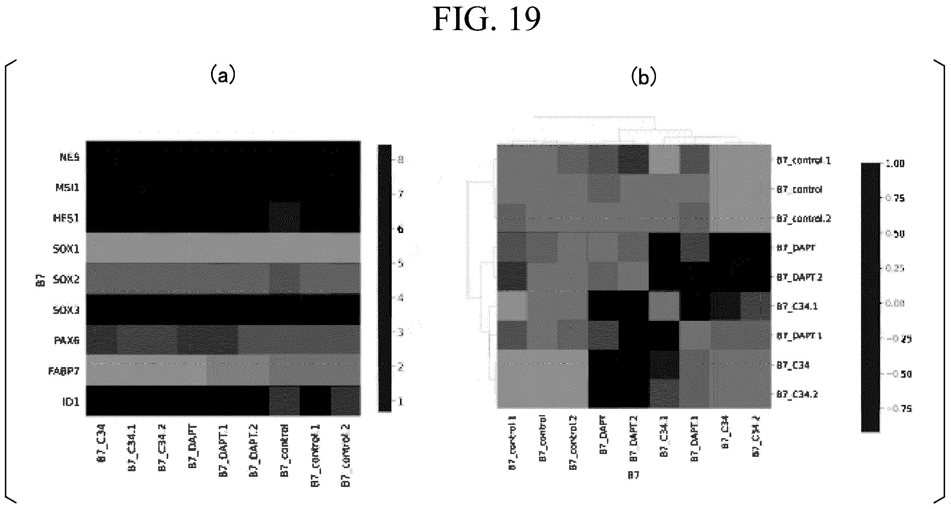

FIGS. 19(a) and 19(b) show views for the neurosphere derived from 201B7 cells, which shows the results obtained by analyzing expression patterns of NESTIN, MUSASHI1, HES1, SOX2, SOX3, PAX6, FABP7, and ID1, which are marker genes of neural stem cells and neural progenitor cells. In FIG. 19, "B7" indicates that it is the result obtained from the neurosphere derived from 201B7 cells, "Control" indicates that it is the result obtained from the control on which neither DAPT nor Compound 34 has been allowed to act, "DAPT" indicates that it is the result obtained by using DAPT, and "C34" indicates that it is the result obtained by using Compound 34.

As a result, it was revealed that, regarding the above-described genes, the gene expression variation pattern after the DAPT treatment and the gene expression variation pattern after the Compound 34 treatment are in a homologous relationship in terms of the clustering. In addition, the same results were also obtained regarding the neurosphere derived from 414C2 cells.

### [Experimental Example 12]

### (Examination of expression of phosphorylated CDC25B in nerve cell)

Neurospheres were treated with DAPT or Compound 34, and the amount of phosphorylated CDC25B after inducing differentiation into nerve cells was examined.

Neurospheres derived from 201B7 cells, which are cells of a human iPS cell line, were prepared in the same manner as in Experimental Example 1. Subsequently, each neurosphere was cultured for 24 hours in the presence of each of DAPT and Compound 34. DAPT and Compound 34 were used at the same concentrations as in Experimental Example 1.

Subsequently, each neurosphere was seeded on a 48-well chamber slide (IWAKI & CO., LTD.) coated with poly-L-ornithine/fibronectin, cultured for 14 days in a culture medium containing no growth factor at 37°C in an environment of 95% air and 5% CO₂, and differentiated into nerve cells.

Subsequently, each of the obtained nerve cells was fixed with 4% paraformaldehyde and subjected to immunostaining of each of βIII-tubulin, which is a nerve cell marker, and phosphorylated CDC25B.

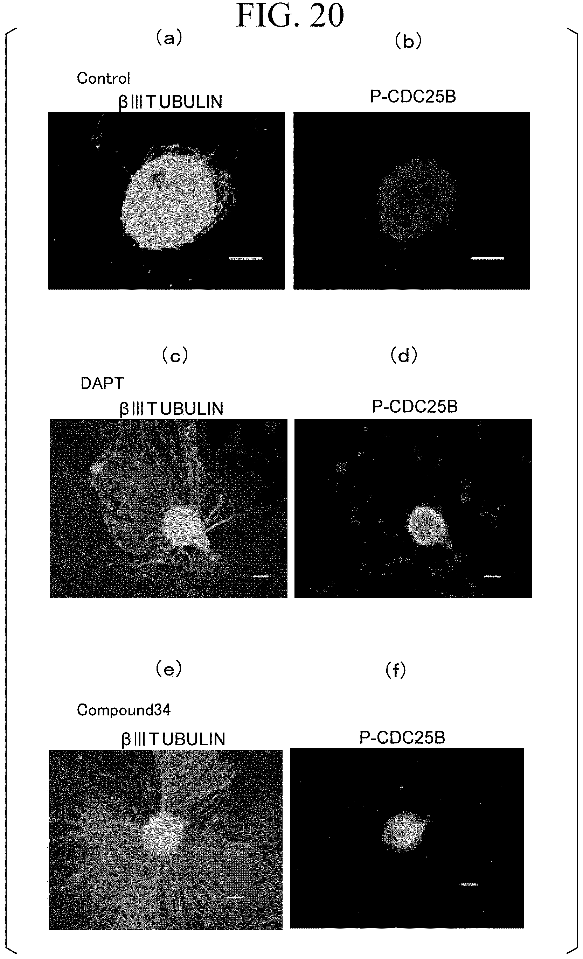

FIGS. 20(a) to 20(f) show fluorescence photomicrographs showing the representative results of immunostaining. The scale bar is 100 µm. FIGS. 20(a) and 20(b) show the results obtained from the control which neither the DAPT treatment nor the Compound 34 treatment has been carried out, FIG. 20(c) and 20(d) show the results obtained by carrying out the DAPT treatment, and FIG. 20(e) and 20(f) show the results obtained by carrying out the Compound 34 treatment. In addition, FIGS. 20(a), 20(c), and 20(e) show the results obtained by immunostaining of βIII-tubulin, and FIG. 20(b), 20(d), and 20(f) show the results obtained by immunostaining of phosphorylated CDC25B.

As a result, it was revealed that in a case where the neurosphere is treated with DAPT or Compound 34, the amount of phosphorylated CDC25B is increased as compared with the untreated case, even after inducing differentiation into nerve cells.

### [Experimental Example 13]

### (Verification 7 of signal cascade associated with neurite outgrowth)

### <<Examination of effect of overexpression of GADD45G on phosphorylation of p38>>

Using a lentiviral vector, GADD45G was overexpressed in neurospheres derived from 201B7 cells and 414C2 cells, which are cells of human iPS cell lines, and the effect on the phosphorylation of p38 was examined.

First, neurospheres derived from 201B7 cells and neurospheres derived from 414C2 cells were each prepared in the same manner as in Experimental Example 1. Subsequently, by using a lentiviral vector, a construct for GADD45G expression was introduced into the neurospheres in each group, and GADD45G was overexpressed. In addition, a group into which an empty lentiviral vector was introduced was prepared as a control. Subsequently, each neurosphere was fixed with paraformaldehyde and subjected to immunostaining of phosphorylated p38 and p38.

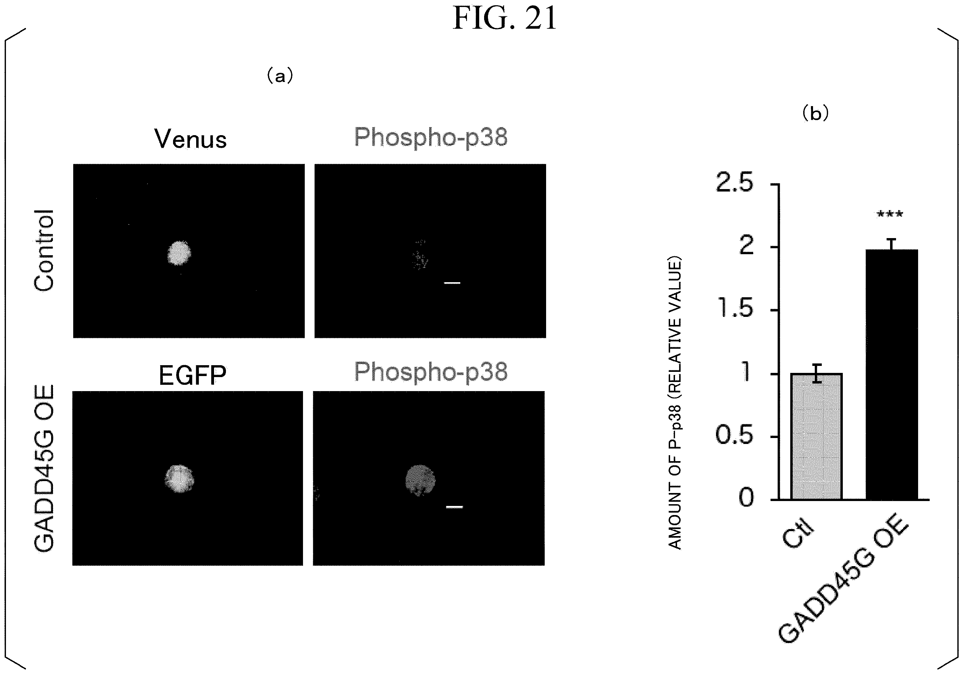

FIG. 21(a) shows fluorescence photomicrographs showing representative results of immunostaining of phosphorylated p38 in the neurosphere derived from 201B7 cells. The scale bar is 100 µm. In FIG. 21(a), "Control" indicates that it is the result obtained from the control neurosphere, "GADD45G OE" indicates that it is the result obtained from the neurosphere overexpressing GADD45G, and "Phospho-p38" indicates that it is the result obtained by detecting phosphorylated p38. "Venus" was used as a reporter of an empty lentiviral vector. In addition, "EGFP" was used as a reporter of a lentiviral vector for GADD45G expression.

FIG. 21(b) shows a graph showing results obtained by quantifying the amount of the phosphorylated p38 in the neurosphere derived from the 201B7 cells in each group. In FIG. 21(b), "Ctl" indicates that it is the result obtained from the control neurosphere, and "GADD45G OE" indicates that it is the result obtained from the neurosphere overexpressing GADD45G. In addition, "***" indicates p < 0.001 (n = 3) and indicates that there is a significant difference.

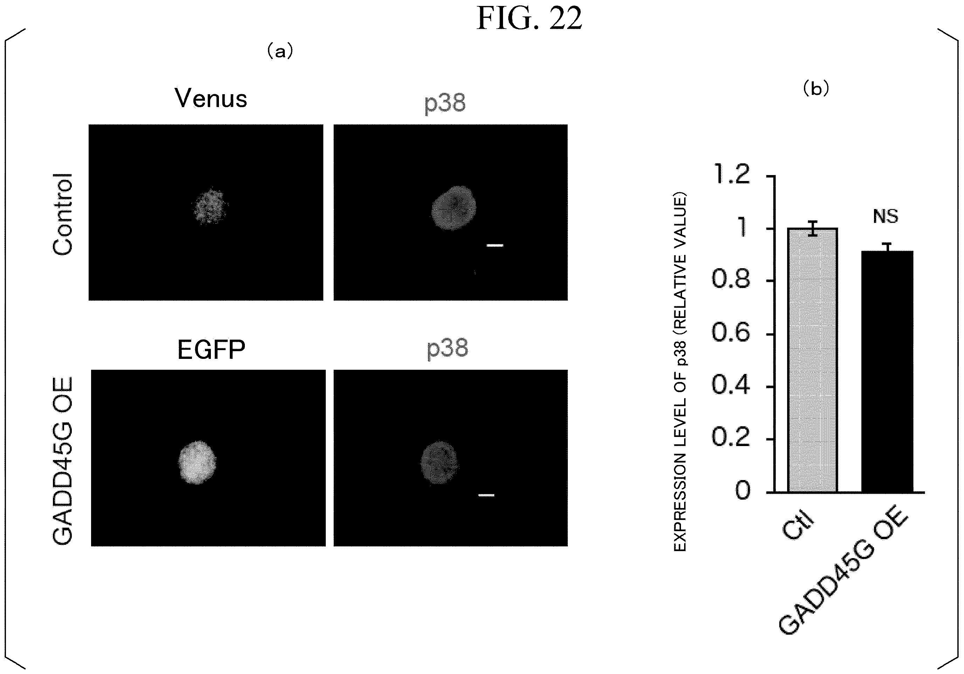

FIG. 22(a) shows fluorescence photomicrographs showing representative results of immunostaining of p38 in the neurosphere derived from 201B7 cells. The scale bar is 100 µm. In FIG. 22(a), "Control" indicates that it is the result obtained from the control neurosphere, "GADD45G OE" indicates that it is the result obtained from the neurosphere overexpressing GADD45G, and "p38" indicates that it is the result obtained by detecting p38. "Venus" was used as a reporter of an empty lentiviral vector. In addition, "EGFP" was used as a reporter of a lentiviral vector for GADD45G expression.

FIG. 22(b) shows a graph showing results obtained by quantifying the amount of p38 in the neurosphere derived from the 201B7 cells in each group. In FIG. 22(b), "Ctl" indicates that it is the result obtained from the control neurosphere, and "GADD45G OE" indicates that it is the result obtained from the neurosphere overexpressing GADD45G. In addition, "NS" indicates that there is no significant difference (n = 3).

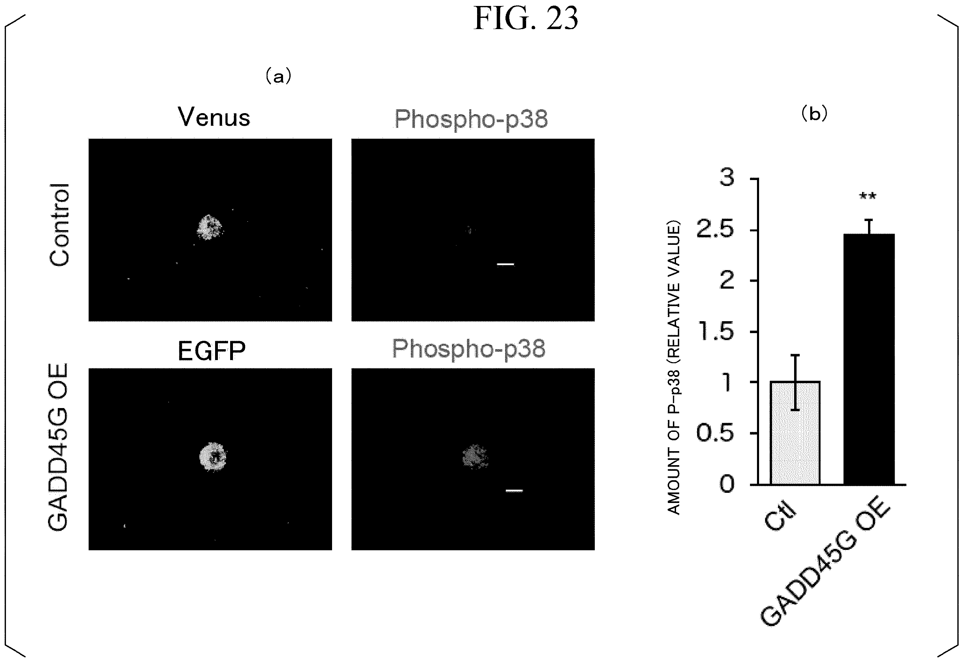

FIG. 23(a) shows fluorescence photomicrographs showing representative results of immunostaining of phosphorylated p38 in a neurosphere derived from 414C2 cells. The scale bar is 100 µm. In FIG. 23(a), "Control" indicates that it is the result obtained from the control neurosphere, "GADD45G OE" indicates that it is the result obtained from the neurosphere overexpressing GADD45G, and "Phospho-p38" indicates that it is the result obtained by detecting phosphorylated p38. "Venus" was used as a reporter of an empty lentiviral vector. In addition, "EGFP" was used as a reporter of a lentiviral vector for GADD45G expression.

FIG. 23(b) shows a graph showing results obtained by quantifying the amount of the phosphorylated p38 in the neurosphere derived from the 414C2 cells in each group. In FIG. 23(b), "Ctl" indicates that it is the result obtained from the control neurosphere, and "GADD45G OE" indicates that it is the result obtained from the neurosphere overexpressing GADD45G. In addition, "**" indicates p < 0.01 (n = 3) and indicates that there is a significant difference.

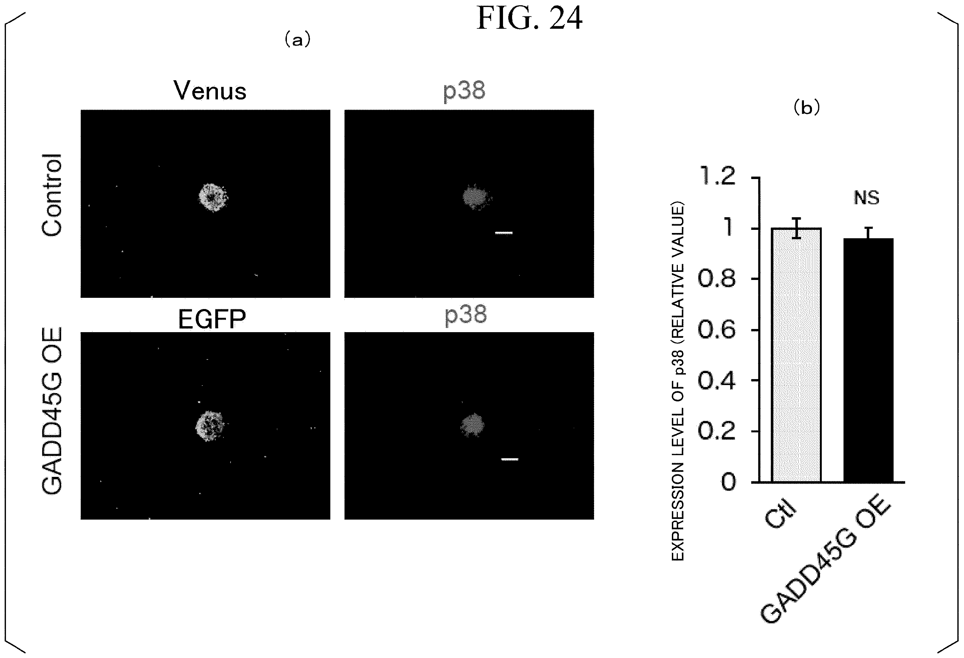

FIG. 24(a) shows fluorescence photomicrographs showing representative results of immunostaining of p38 in a neurosphere derived from 414C2 cells. The scale bar is 100 µm. In FIG. 24(a), "Control" indicates that it is the result obtained from the control neurosphere, "GADD45G OE" indicates that it is the result obtained from the neurosphere overexpressing GADD45G, and "p38" indicates that it is the result obtained by detecting p38. "Venus" was used as a reporter of an empty lentiviral vector. In addition, "EGFP" was used as a reporter of a lentiviral vector for GADD45G expression.

FIG. 24(b) shows a graph showing results obtained by quantifying the amount of p38 in the neurosphere derived from the 414C2 cells in each group. In FIG. 24(b), "Ctl" indicates that it is the result obtained from the control neurosphere, and "GADD45G OE" indicates that it is the result obtained from the neurosphere overexpressing GADD45G. In addition, "NS" indicates that there is no significant difference (n = 3).

As a result, it was revealed that the amount of phosphorylated p38 in the neurosphere is significantly increased by the overexpression of GADD45G, but the expression level of p38 is not increased.

### [Experimental Example 14]

### (Verification 8 of signal cascade associated with neurite outgrowth)

### <<Examination of effect of overexpression of GADD45G on neurite outgrowth>>

Using a lentiviral vector, GADD45G was overexpressed in neurospheres derived from 201B7 cells and 414C2 cells, which are cells of human iPS cell lines, and the effect on the neurite outgrowth was examined.

First, neurospheres derived from 201B7 cells and neurospheres derived from 414C2 cells were each prepared in the same manner as in Experimental Example 1. Subsequently, by using a lentiviral vector, a construct for GADD45G expression was introduced into the neurospheres in each group, and GADD45G was overexpressed. In addition, a group into which an empty lentiviral vector was introduced was prepared as a control.

Subsequently, each neurosphere was seeded on a 48-well chamber slide (IWAKI & CO., LTD.) coated with poly-L-ornithine/fibronectin, cultured for 14 days in a culture medium containing no growth factor at 37°C in an environment of 95% air and 5% CO₂, and differentiated into nerve cells.

Subsequently, each of the obtained nerve cells was fixed with 4% paraformaldehyde in 0.1 M PBS, the Tau protein was immunostained, and the length of the neurite was measured.

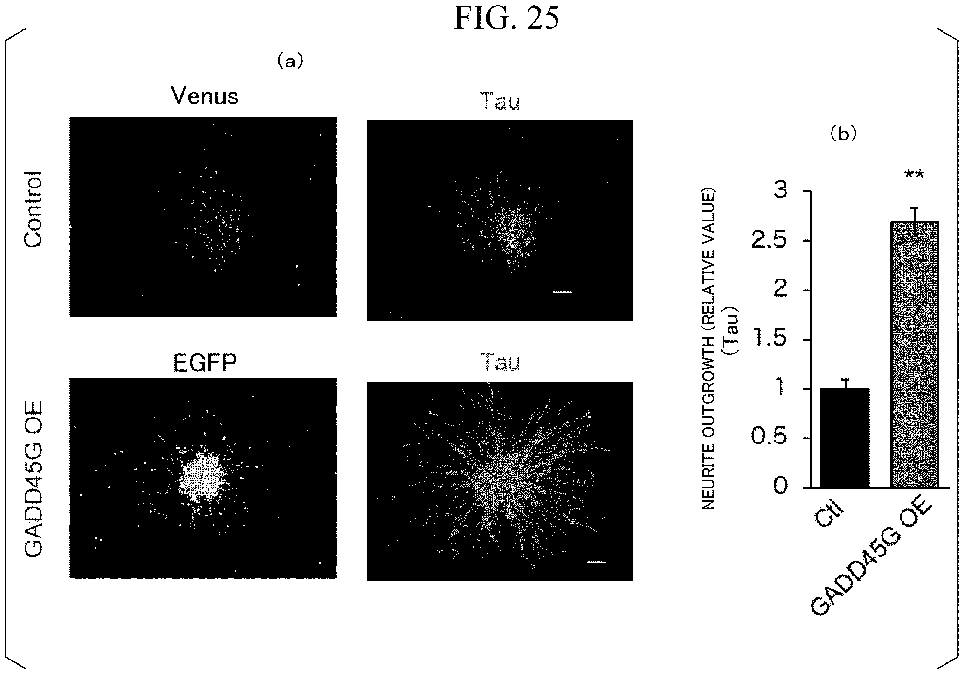

FIG. 25(a) shows fluorescence photomicrographs showing representative results of immunostaining of a Tau protein in nerve cells derived from 201B7 cells. The scale bar is 100 µm. In FIG. 25(a), "Control" indicates that it is the result obtained from the control neurosphere, and "GADD45G OE" indicates that it is the result obtained from the neurosphere overexpressing GADD45G. "Venus" was used as a reporter of an empty lentiviral vector. In addition, "EGFP" was used as a reporter of a lentiviral vector for GADD45G expression.

FIG. 25(b) shows a graph showing results obtained by quantifying the neurite length of nerve cells derived from the 201B7 cells in each group. In FIG. 25(b), "Ctl" indicates the result obtained from the control nerve cells, and "GADD45G OE" indicates that it is the result obtained from the nerve cells overexpressing GADD45G. In addition, "**" indicates p < 0.01 (n = 3) and indicates that there is a significant difference.

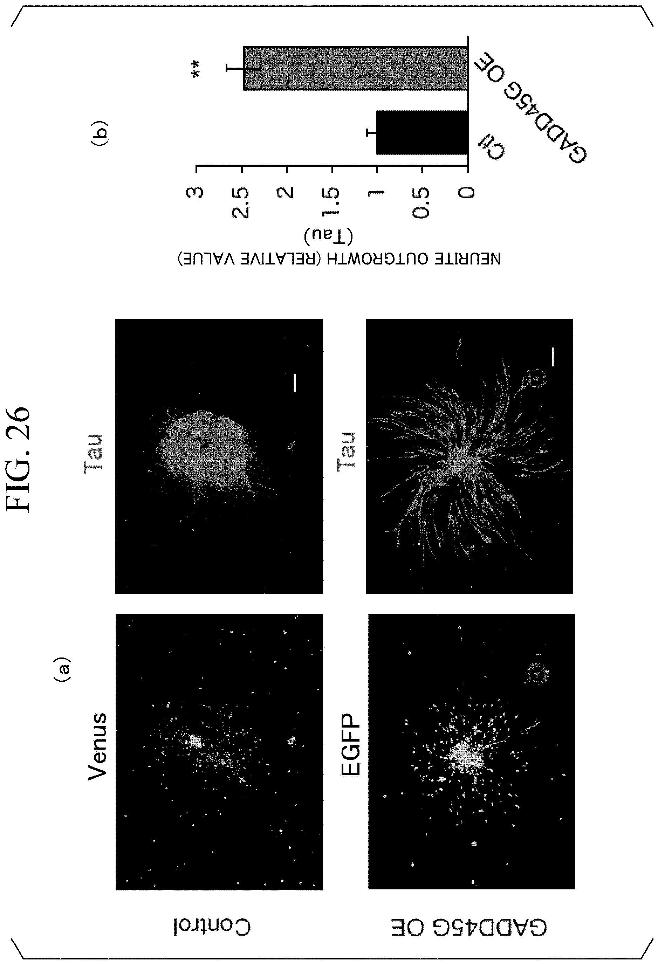

FIG. 26(a) shows fluorescence photomicrographs showing representative results of immunostaining of the Tau protein in nerve cells derived from 414C2 cells. The scale bar is 100 µm. In FIG. 26(a), "Control" indicates that it is the result obtained from the control neurosphere, and "GADD45G OE" indicates that it is the result obtained from the neurosphere overexpressing GADD45G. "Venus" was used as a reporter of an empty lentiviral vector. In addition, "EGFP" was used as a reporter of a lentiviral vector for GADD45G expression.

FIG. 26(b) shows a graph showing results obtained by quantifying the neurite length of nerve cells derived from the 414C2 cells in each group. In FIG. 26(b), "Ctl" indicates the result obtained from the control nerve cells, and "GADD45G OE" indicates that it is the result obtained from the nerve cells overexpressing GADD45G. In addition, "**" indicates p < 0.01 (n = 3) and indicates that there is a significant difference.

As a result, it was revealed that in a case where GADD45G is overexpressed in the neurosphere, neurite outgrowth is significantly promoted in a case where the neurosphere is induced to differentiate into nerve cells.

In Experimental Examples 9 and 10, It was confirmed that in a case where RK-682 is administered into the neurosphere, the signal of p38/CDC25B is enhanced, and the neurite outgrowth is promoted. From the present experimental examples, it was revealed that the same result can be obtained even in a case where the expression of the more upstream GADD45G is increased with a lentivirus.

From the above results, it has confirmed that the signal cascade shown in FIG. 10 is correct in terms of the compound administration and in terms of the genetic engineering.

### [Industrial Applicability]

According to the present invention, it is possible to provide a technique for promoting neurite outgrowth of nerve cells derived from a pluripotent stem cell.

## Claims

1. A neurite outgrowth promotion kit, comprising:
a γ-secretase inhibitor; or
a GADD45G expression vector,
wherein the neurite outgrowth promotion kit is used for promoting neurite outgrowth of nerve cells as compared with a control, by acting on a neurosphere to induce differentiation of the neurosphere into nerve cells.

2. The neurite outgrowth promotion kit according to Claim 1,
wherein the γ-secretase inhibitor is Compound 34 or DAPT.

3. The neurite outgrowth promotion kit according to Claim 1, further comprising:
a dephosphorylation inhibitor of phosphorylated p38 MAPK.

4. The neurite outgrowth promotion kit according to Claim 3,
wherein the dephosphorylation inhibitor of the phosphorylated p38 MAPK is RK-682.

5. A method for producing a neurosphere for treatment of spinal cord injury, the method comprising:
a step of using the neurite outgrowth promotion kit according to any one of Claims 1 to 4 on a neurosphere derived from pluripotent stem cells.

6. A neurosphere for treatment of spinal cord injury,
wherein an expression level of GADD45G or an amount of phosphorylated CDC25B is increased as compared with a control.

7. A method for selecting a neurosphere for treatment of spinal cord injury, the method comprising:
a step of measuring an expression level of GADD45G or an amount of phosphorylated CDC25B in a neurosphere,
wherein a case where the measured expression level of the GADD45G or the measured amount of the phosphorylated CDC25B is increased as compared with a control indicates that the neurosphere is suitable for treatment of spinal cord injury.

8. A method for screening a neurite outgrowth-promoting agent, comprising:
a step of culturing a neurosphere in a presence of a test substance; and
a step of measuring an expression level of GADD45G or an amount of phosphorylated CDC25B in the neurosphere,
wherein a case where the expression level of the GADD45G or the amount of the phosphorylated CDC25B is increased as compared with a case where the test substance is not present indicates that the test substance is a neurite outgrowth-promoting agent.
